# FASCICULE DE BREVET EUROPEEN

(11) **EP 2 068 953 B1**
(45) Date de publication et mention de la délivrance du brevet: **30.11.2011**
(21) Numéro de dépôt: 07820627.3
(22) Date de dépôt: 26.09.2007
(51) Int. Cl.: A61L 27/14, A61L 27/32, A61L 27/50, A61L 27/18

(54) **PROCEDE DE SABLAGE DE POLYMERES BIOCOMPATIBLES**
SANDSTRAHLVERFAHREN MIT BIOKOMPATIBLEN POLYMEREN
SAND-BLASTING METHOD USING BIOCOMPATIBLE POLYMERS

(30) Priorité: 26.09.2006 FR 0608436
(43) Date de publication de la demande: 17.06.2009
(73) Titulaire: Biomatlante, 44360 Vigneux de Bretagne (FR)
(72) Inventeur: BOURGES, Xavier, 01140 Mogneneins (FR); GOBIN, Chantal, F-44360 Vigneux de Bretagne (FR)
(74) Mandataire: Warcoin, Jacques
(86) Numéro de dépôt international: PCT/EP2007/060232
(87) Numéro de publication internationale: WO 2008/037751

(56) Documents cités:
- EP-A- 1 348 454
- EP-A- 1 674 051
- WO-A-03/047470
- WO-A1-02/102431
- FR-A- 2 865 939
- US-A- 5 251 468

## Description

La présente invention concerne la préparation d'implants biocompatibles. Plus particulièrement, elle a pour objet une méthode de modification de la surface de polymères, résorbables ou non, et utilisés en tant qu'implants médicaux. Elle permet d'améliorer la bioactivité et/ou l'ancrage de ces implants au contact des tissus vivants du site d'implantation.

L'invention consiste à sabler ces implants à l'aide de particules abrasives de type phosphocalcique résorbables. Les particules peuvent être soit laissées à la surface de l'implant, soit dissoutes à l'aide d'un acide selon le degré de pureté de la surface recherché et selon l'effet biologique éventuellement induit par les particules intégrées dans la surface.

Le sablage d'implants médicaux est très développé, autant en implantologie dentaire pour les implants en titane, ou en chirurgie orthopédique tels que les prothèses de hanche, les ostéosynthèses.

Il a été montré que la micro géométrie et la rugosité de la surface des implants jouaient un rôle prédominant dans leur intégration au niveau des tissus mous ou durs. La microrugosité a une influence sur la stabilité mécanique des implants, et sur leur énergie de surface, et modifie leur mouillabilité. En milieu osseux, l'augmentation de la rugosité augmente la surface des implants en titane donc le contact osseux, et parallèlement les propriétés mécaniques et d'ancrage de ceux-ci.

La demande de brevet FR 04 01151 (publiée sous le numéro 2 865 939) décrit l'emploi d'une poudre abrasive, composée de phosphate de calcium, afin de modifier la surface d'implants métalliques qui sont ensuite recouverts d'un hydrogel silanisé. Dans cette demande, le sablage est destiné à améliorer le greffage du gel à la surface du métal, qui n'est pas en contact direct avec les cellules osseuses.

*In vitro*, la prolifération et la différentiation des ostéoblastes (les cellules à l'origine de la matrice extracellulaire qui se minéralise par la suite) sont affectées par l'état de surface de l'implant. Les surfaces sablées présentent des irrégularités, qui favorisent l'ostéointégration. Les cellules cultivées sur ce genre de surface secrètent une matrice extra cellulaire plus importante, expriment plus facilement la phosphatase alcaline, et se différencient plus aisément en ostéoblastes.

Les différents médias de sablage utilisés aujourd'hui sont des matériaux de haute dureté telle que l'alumine, la silice ou l'oxyde de titane. Le problème suscité par ce genre de produit réside dans le fait qu'ils sont difficiles à extraire des surfaces traitées. Des procédés physico-chimiques souvent délicats à mettre en oeuvre sont généralement nécessaires afin d'assurer un nettoyage correct des surfaces traitées, comme par exemple la dissolution du média à laide d'acide fluorhydrique. Des particules de ces médias de sablage, mais aussi des complexes ioniques peuvent rester à la surface, ne laissant pas celle-ci dans un état suffisamment propre ou propice au contact osseux. En effet les particules de silice ou d'alumine peuvent entraîner la formation d'un tissu conjonctif et non minéralisé qui va engendrer un mauvais contact osseux. Les particules de silice peuvent aussi induire des phénomènes de réaction à corps étrangers et d'ostéolyse du tissu osseux hôte ou néoformé, avant la libération de ces particules toxiques.

Les conditions fortement acides nécessaires à l'élimination complète de la silice ou de l'alumine, ne permettent pas le recours au sablage sur les prothèses en matériaux polymériques, car ces derniers sont plus sensibles aux acides que les métaux. Or les prothèses métalliques, comme le titane, présentent une dureté qui ne permet pas leur utilisation dans de nombreuses indications chirurgicales. C'est le cas, par exemple, des cages de fusion pour l'arthrodèse vertébrale, qui s'enfoncent dans la vertèbre quand elles sont constituées de titane et provoquent dans certains cas des impact ions.

Des pièces en matériaux plastiques sont couramment implantées, mais leur bioactivité est plus ou moins importante selon leur structure chimique et selon l'état de leur surface. La plupart des matériaux plastiques sont obtenus par moulage ou extrusion. Ils présentent généralement des états de surface extrêmement lisses, c'est-à-dire des rugosités de l'ordre de 0,5 à 0,2 Ra seulement.

Le polyarylétheréthercétone (PEEK) est un polymère inerte couramment utilisé pour des pièces anatomiques médicales. Il est toutefois connu qu'il est impossible de faire pousser de l'os sur ce matériau, qui n'engendre pas un contact direct avec l'os et provoque des fibroses. Il ne présente en effet pas une surface adhérente pour les cellules, en raison notamment de sa nature chimique. Celle-ci offre de plus peu de possibilités de modification par traitement chimique. Ainsi, le PEEK a jusqu'à présent été utilisé uniquement comme pièce de maintien, mais jamais pour établir un contact osseux intime.

Les auteurs de la présente invention ont trouvé le moyen résoudre ce problème, grâce à un procédé permettant de modifier la surface du PEEK, à l'aide d'un média de sablage d'origine phosphocalcique, qui peut être ensuite éliminé à l'aide d'un lavage à l'acide faible ou à l'acide fort dilué. La plupart des plastiques biocompatibles sont en effet dégradés par les acides forts, tels que l'acide fluorhydrique, qui sont couramment utilisés pour dissoudre les médias de sablage type alumine ou silice.

La présente invention permet donc d'obtenir un implant en PEEK dont la surface est à la fois rugueuse et très propre.

Plus précisément, l'invention concerne un procédé de modification de l'état de surface d'un implant en polyarylétheréthercétone, pour favoriser son ostéoconduction et son ostéointégration en chirurgie osseuse, impliquant le sablage dudit implant par des particules abrasives de phosphate de calcium.

Ce procédé permet pour la première fois d'augmenter la surface du matériau polymérique biocompatible et donc d'augmenter le contact avec les tissus du site d'implantation. Il permet également un meilleur ancrage de ce type de matériaux ainsi qu'une une meilleure bioactivité, en recrutant beaucoup plus de cellules à leur surface.

Le media de sablage peut avantageusement être composé de différentes particules de phosphate de calcium obtenues naturellement ou par frittage, choisi dans la liste comprenant l'hydroxyapatite (Ca₁₀(PO₄)₆(OH)₂), le béta-phosphate de tricalcium (β-Ca₃(PO₄)₂), l'alpha-phosphate de tricalcium (α-Ca₃(PO₄)₂), le phosphate tetracalcique (Ca₄(PO₄)₂O₂), le phosphate octocalcique (Ca₈H₂(PO₄)₆, 5H₂O) ainsi que leurs mélanges.

Avantageusement, les particules abrasives comprennent de l'hydroxyapatite et au moins un phosphate de calcium choisi dans la liste comprenant le béta-phosphate de tricalcium, l'alpha phosphate de tricalcium, le phosphate tetracalcique et le phosphate octocalcique .

Avantageusement, la proportion d'hydroxyapatite dans le mélange est comprise entre 60 et 40% en poids.

Plus avantageusement encore, les particules abrasives comprennent de l'hydroxyapatite et du β-phosphate de tricalcium.

Avantageusement le ratio hydroxyapatite/β-phosphate de tricalcium est compris entre 2/3 et 1 en poids.

Plus avantageusement, les particules abrasives comprennent en poids 85% d'hydroxyapatite et 15% de β-phosphate de tricalcium.

Avantageusement encore, les particules abrasives ont une taille comprise entre 150 et 700 µm, de façon plus avantageuse entre 300 et 700 µm.

De façon avantageuse, les particules abrasives ont une dureté Vickers comprise entre 450 et 1200, plus avantageusement encore entre 500 et 1000.

De façon encore plus avantageuse, les particules abrasives comprennent en poids 85% d'hydroxyapatite et 15% de β-phosphate de tricalcium de taille comprise entre 300 et 700 µm et de dureté Vickers comprise entre 450 et 1200, de préférence égale à 550.

Afin d'utiliser des telles particules en tant que média de sablage à usage biomédical, des analyses en teneur de métaux lourds sont nécessaires et les résultats doivent être conformes aux normes en vigueur.

Aussi les particules doivent présenter une dureté suffisante afin de conserver leur pouvoir abrasif.

Les tailles de ces particules abrasives peuvent être variables selon la rugosité finale choisie. En effet, plus les particules sont petites, plus la rugosité obtenue est faible et uniforme (avec un effet de brossage). *A contrario*, des particules de taille supérieure engendrent une rugosité et une hétérogénéité du relief de la surface plus importante.

Ces phosphates de calcium ont avantageusement une composition identique à celle de l'os et leur efficacité en tant que substituts osseux et a été largement démontrée.

L'avantage prédominant de ce type de média est qu'il est particulièrement soluble, et ceci très rapidement, dans les acides faibles ou les acides forts dilués.

Les particules de phosphate de calcium résiduelles peuvent être rincées de la surface du polymère à l'aide d'une solution aqueuse d'acide faible ou d'acide fort dilué.

Avantageusement, ces solutions d'acide peuvent être l'acide nitrique dilué à 26% ou l'acide acétique dilué à 15%.

Ces solutions sont suffisamment acides pour débarrasser la surface en totalité du phosphate de calcium, sans pour autant dégrader celle-ci.

L'invention a donc également pour objet un procédé tel que défini ci-dessus, qui comprend en outre une étape subséquente de lavage, avantageusement par une solution aqueuse acide.

La solubilité en milieu acide d'un média de sablage composé de 85% d'hydroxyapatite et 15% de béta phosphate de tricalcium, dont la taille de particules est comprise entre 300 et 700 µm et de dureté Vickers de 550 a été étudiée. 5 grammes de média ont été suspendus dans 100 ml de solution d'acide nitrique à 15 %. Après 8 minutes, le taux d'insolubles était en moyenne de 0,046 %. Des analyses par spectroscopie EDX (spectroscopie de dispersion d'énergie des rayons X) ont montré que ces insolubles étaient composés principalement de phosphates de calcium non identifiables et de traces de silicium et de magnésium.

Des essais de sablage à base de phosphate de calcium sur des pièces en titane ont montré qu'aucun résidu solide ne se présentait à la surface du matériau après nettoyage à l'acide.

Dans le cas où le PEEK a subi un sablage, il est possible ensuite de modifier sa surface en le soumettant à un traitement de type plasmatique. Il peut consister à projeter des particules d'hydroxyapatite ou de BCP chauffées à haute température au moyen d'une torche à plasma, afin de recouvrir l'implant d'une couche uniforme et lisse de phosphate de calcium. Le fait d'avoir une surface sablée très propre, c'est-à-dire dépourvue de tout élément solide propre au média de sablage, permet d'obtenir un traitement final plus efficace qu'avec une surface lisse.

On entend par surface lisse ou usinée une surface dont la rugosité est inférieure à 0,5 Ra.

Avantageusement, il est aussi possible de laisser les particules de phosphate de calcium à la surface du polymère. Ceci a pour effet de favoriser l'ostéoconduction du matériau implanté.

Après l'étape de lavage, l'implant selon l'invention présente l'avantage de ne pas libérer de particules de PEEK.

Les implants abrasés selon l'invention peuvent être utilisés en chirurgie orthopédique, en tant que composé d'articulation, pièces anatomiques pour des vaisseaux sanguins, membrane pour des tissus mous, membrane de soutien et de guidage de la cicatrisation, ostéosynthèse et plus particulièrement dans les cages de fusion pour arthrodèses vertébrales.

Le procédé selon l'invention peut avantageusement être mis en oeuvre sur des inserts ou des vis d'interférence à base d'acide polylactique.

Le procédé selon l'invention peut avantageusement être mis en oeuvre sur des vis, des plaques et toute ostéosynthèse en PEEK servant pour la chirurgie maxillofaciale, traumatologique, orthopédique et plus généralement toute chirurgie osseuse, ostéoarticulaire ou tissulaire non calcifiée.

Les implantations *in vivo* de ces matériaux ont montré que leur bioactivité est supérieure à celle d'un matériau de surface usinée, c'est-à-dire lisse.

L'invention a donc également pour objet un implant pour chirurgie osseuse en polyarylétheréthercétone susceptible d'être préparé par le procédé selon l'invention.

L'implant selon l'invention présente avantageusement une rugosité supérieure ou égale à 0,5 Ra, avantageusement comprise entre 0,5 et 10 Ra.

Avantageusement, la surface de l'implant est très propre, c'est-à-dire qu'elle est dépourvue de particules de phosphate de calcium et de particules de polyarylétheréthercétone libérées.

L'exemple suivant de mise en oeuvre de l'invention est donné à titre d'illustration et n'a aucun caractère limitatif

### EXEMPLE

Des implants en PEEK ont été soumis au procédé selon l'invention afin d'éprouver leur stabilité et leur osteointégration : ils ont été sablés avec un média de sablage constitué de phosphates de calcium, lavés et implantés en site osseux chez des lapins. Les résultats ont été comparés à ceux obtenus avec des implants non sablés.

### Matériels et méthodes

Le PEEK utilisé pour ces implantations est de type commercial Optima (Invibio, Lancashire, Grande Bretagne). 40 cylindres de 6 mm de diamètre et 8 mm de longueur ont été réalisés à partir d'une tige extrudée.

Le média de sablage est un phosphate de calcium biphasé (BCP : 85% hydroxyapatite, 15% beta phosphate de tricalcium) fabriqué par Biomatlante (Vigneux de Bretagne, France). Sa dureté Vickers a été calculée à l'aide d'un duromètre ; sa valeur est de 510.

20 implants sont gardés sans sablage (surfaces lisses), et 20 sont sablés, à l'aide de particules de BCP (40-80 mesh, soit de taille comprise entre 178 et 422 µm), et d'une sableuse de type Clemco PUL 111D, ayant une buse de sablage de 6 mm de diamètre.

Après sablage, les implants sont nettoyés dans une solution d'acide nitrique à 26% pendant 1 heure dans un bain à ultrasons, rincés 3 fois l'aide d'eau déionisée, et ensuite séchés à l'étuve. Les implants de PEEK sont stérilisés à l'autoclave 121°C pendant 20 minutes.

6 implants furent choisis aléatoirement afin de mesurer leur rugosité à l'aide d'un profilomètre Surftest SJ-301 (Mitutoyo, Tokyo, Japon).

La propreté de la surface obtenue a été vérifiée au microscope électronique à balayage.

Les cylindres ont ensuite été implantés au niveau de l'épiphyse fémorale de lapins New Zealand. Les lapins sont sacrifiés par injection après six et douze semaines. Un double marquage à la tétracycline est réalisé 10 jours avant le sacrifice des animaux. Les implants sont déshydratés et inclus dans la résine pour observations et analyses : micro scanner, microscope à lumière polarisée, et microscope électronique à balayage.

### Résultats

Les mesures de rugosité ont montré que le PEEK sorti d'usine avait une rugosité de 0,3 Ra ± 0,1, alors que le PEEK sablé présentait une rugosité de 3,2 ± 0,3 Ra. Cette rugosité peut même être augmentée jusqu'à 8 Ra. Une analyse EDX des implants n'a pas montré la présence de calcium ou de phosphore, signifiant ainsi que la surface était parfaitement propre.

Après six et douze semaines, le PEEK non sablé et le PEEK sablé sont correctement osteointégrés. Aucune réaction inflammatoire liée au sablage n'est observée.

Après douze semaines la densité osseuse apparaît similaire entre les deux groupes d'implants, et un remodelage osseux est observé.

Cependant, l'architecture osseuse apparaît différente pour les deux groupes. La repousse osseuse est plus intime à 6 semaines sur les surfaces sablées que sur les surfaces lisses (meilleure ostéointégration), sans doute liée à une meilleure stabilité de l'implant. L'ostéoconduction est plus importante à 6 et 12 semaines pour les implants sablés. Sur les deux types de surfaces, une coque osseuse recouvrant l'implant est observée. Le contact de cette coque osseuse lamellaire est plus intime avec la surface sablée. L'architecture osseuse est aussi mieux organisée sur la surface sablée, qui présente beaucoup plus de ponts osseux perpendiculaires. Aussi, une analyse à l'aide d'un microscanner révèle beaucoup plus d'artéfacts entre les implants non sablés et l'os néoformé.

## Revendications

1. Procédé de modification de l'état de surface d'un implant en polyarylétheréthercétone, pour favoriser son ostéoconduction et son ostéointégration en chirurgie osseuse, impliquant le sablage dudit implant par des particules abrasives de phosphate de calcium.

2. Procédé selon la revendication 1, **caractérisé en ce que** les particules abrasives de phosphate de calcium peuvent être choisies dans la liste comprenant l'hydroxyapatite, le β-phosphate de tricalcium, le α-phosphate de tricalcium, le phosphate tetracalcique, le phosphate octocalcique et les mélanges des composés ci-dessus.

3. Procédé selon l'une quelconque des revendications précédentes, **caractérisé en ce que** les particules abrasives de phosphate de calcium comprennent de l'hydroxyapatite et du β-phosphate de tricalcium.

4. Procédé selon l'une quelconque des revendications précédentes, **caractérisé en ce que** les particules abrasives de phosphate de calcium ont une taille comprise entre 150 et 700 µm, de préférence entre 300 et 700 µm.

5. Procédé selon l'une quelconque des revendications précédentes, **caractérisé en ce que** les particules abrasives de phosphate de calcium ont une dureté Vickers comprise entre 450 et 1200, de préférence entre 500 et 1000.

6. Procédé selon l'une quelconque des revendications précédentes, **caractérisé en ce que** les particules abrasives de phosphate de calcium comprennent en poids 85% d'hydroxyapatite et 15% de β-phosphate de tricalcium de taille comprise entre 300 et 700 µm et de dureté Vickers comprise entre 450 et 1200.

7. Procédé selon l'une quelconque des revendications précédentes, **caractérisé en ce qu'**il comprend une étape subséquente de lavage, de préférence par une solution aqueuse acide.

8. Procédé selon l'une quelconque des revendications précédentes, **caractérisé en ce qu'**il comprend une étape subséquente de modification de la surface de l'implant par un traitement de type plasmatique.

9. Implant pour chirurgie osseuse en polyarylétheréthercétone, **caractérisé en ce qu'**il est susceptible d'être préparé par un procédé selon l'une quelconque des revendications précédentes et **en ce qu'**il présente une rugosité supérieure ou égale à 0,5 Ra, de préférence comprise entre 0,5 et 10 Ra.

10. Implant selon la revendication 9, **caractérisé en ce que** sa surface est dépourvue de particules de phosphate de calcium et de particules de polyarylétheréthercétone libérées.

## Claims

1. A method for modifying the surface condition of an implant in polyaryletheretherketone, in order to promote its osteoconduction and its osteointegration in bone surgery, involving the sand-blasting of said implant with abrasive calcium phosphate particles.

2. The method according to claim 1, **characterized in that** the abrasive calcium phosphate particles may be selected from the list comprising hydroxyapatite, tricalcium β-phosphate, tricalcium α-phosphate, tetracalcium phosphate, octacalcium phosphate and mixtures of the compounds above.

3. The method according to any of the preceding claims, **characterized in that** the abrasive calcium phosphate particles comprise hydroxyapatite and tricalcium β-phosphate.

4. The method according to any of the preceding claims, **characterized in that** the abrasive calcium phosphate particles have a size in the range of 150 and 700 µm, preferably of 300 and 700 µm.

5. The method according to any of the preceding claims, **characterized in that** the abrasive calcium phosphate particles have a Vickers hardness in the range of 450 and 1,200, preferably of 500 and 1,000.

6. The method according to any of the preceding claims, **characterized in that** the abrasive calcium phosphate particles comprise 85% of hydroxyapatite and 15% of tricalcium β-phosphate by weight with a size in the range of 300 and 700 µm and a Vickers hardness in the range of 450 and 1,200.

7. The method according to any of the preceding claims, **characterized in that** it comprises a subsequent washing step, preferably with an acidic aqueous solution.

8. The method according to any of the preceding claims, **characterized in that** it comprises a subsequent step for modifying the surface of the implant with a plasma type treatment.

9. An implant for bone surgery in polyaryletheretherketone, **characterized in that** it may be prepared by a method according to any of the preceding claims and **in that** it has a roughness larger than or equal to 0.5 Ra, preferably in the range of 0.5 and 10 Ra.

10. The implant according to claim 9, **characterized in that** its surface is free of calcium phosphate particles and of released polyaryletheretherketone particles.

## Patentansprüche

1. Verfahren zur Änderung des Oberflächenzustands eines Implantats aus Polyaryletheretherketon, um dessen knochenchirurgische Osteokonduktion und Osteointegration zu begünstigen, welches das Sandstrahlen des Implantats mittels Calciumphosphat-Schleifpartikel umfasst.

2. Verfahren nach Anspruch 1, **dadurch gekennzeichnet, dass** die Calciumphosphat-Schleifpartikel aus der folgenden Liste ausgewählt werden können: Hydroxyapatit, Tricalcium-β-phosphat, Tricalcium-α-phosphat, Tetracalcium-Phosphat, Octocalcium-Phosphat und Gemische der obigen Komponenten.

3. Verfahren nach einem der vorstehenden Ansprüche, **dadurch gekennzeichnet, dass** die Calciumphosphat-Schleifpartikel Hydroxyapatit und Tricalcium-β-phosphat aufweisen.

4. Verfahren nach einem der vorstehenden Ansprüche, **dadurch gekennzeichnet, dass** die Calciumphosphat-Schleifpartikel eine Größe zwischen 150 und 700µm, vorzugsweise zwischen 300 und 700µm, aufweisen.

5. Verfahren nach einem der vorstehenden Ansprüche, **dadurch gekennzeichnet, dass** die Calciumphosphat-Schleifpartikel eine Vickers-Härte zwischen 450 und 1200, vorzugsweise zwischen 500 und 1000, haben.

6. Verfahren nach einem der vorstehenden Ansprüche, **dadurch gekennzeichnet, dass** die Calciumphosphat-Schleifpartikel 85 Gewichtsprozent Hydroxyapatit und 15 Gewichtsprozent Tricalcium-β-phosphat einer Größe zwischen 300 und 700µm und einer Vickers-Härte zwischen 450 und 1200 aufweisen.

7. Verfahren nach einem der vorstehenden Ansprüche, **dadurch gekennzeichnet, dass** es einen nachfolgenden Waschschritt, vorzugsweise mit einer sauren wässrigen Lösung aufweist.

8. Verfahren nach einem der vorstehenden Ansprüche, **dadurch gekennzeichnet, dass** es einen nachfolgenden Schritt der Änderung der Oberfläche des Implantats durch eine Plasmabehandlung aufweist.

9. Knochenchirurgisches Implantat aus Polyaryletherether-keton, **dadurch gekennzeichnet, dass** es durch ein Verfahren nach einem der vorstehenden Ansprüche herstellbar ist, und dadurch, dass es eine Rauheit von größer oder gleich 0,5Ra, vorzugsweise zwischen 0,5 und 10Ra, aufweist.

10. Implantat nach Anspruch 9, **dadurch gekennzeichnet, dass** seine Oberfläche keine Calciumphosphat-Partikel und freigesetzte Polyaryletheretherketon-Partikel aufweist.
